**Europäisches Patentamt**

**European Patent Office**

**Office européen des brevets**

(19)

(11) **EP 0 865 787 B1**

(12) **EUROPEAN PATENT SPECIFICATION**

(45) Date of publication and mention
of the grant of the patent:
**07.08.2002   Bulletin 2002/32**

(51) Int Cl.⁷: $A61K\ 9/70$, $A61K\ 9/12$

(21) Application number: **98302001.7**

(22) Date of filing: **17.03.1998**

(54) **Method of making controlled release compositions**

Verfahren zur Herstellung von Mitteln mit gesteuerter Freisetzung

Procédé de production de compositions à libération contrôlée

(84) Designated Contracting States:
**DE FR GB**

(30) Priority: **20.03.1997   FR 9703420**

(43) Date of publication of application:
**23.09.1998   Bulletin 1998/39**

(73) Proprietor: **Dow Corning France S.A.**
**06904 Sophia Antipolis (FR)**

(72) Inventors:
• **Aguadisch, Louis Michel Jacques**
**925 Route de Cannes, 06560 Valbonne (FR)**
• **Dalle, Frederic**
**1950 Kraainem (BE)**
• **Stalet, Gilles**
**06250 Mougins (FR)**

(74) Representative: **Vandamme, Luc Johan Roger**
**Dow Corning Limited,**
**Cardiff Road**
**Barry, South Glamorgan CF63 2YL, Wales (GB)**

(56) References cited:
**DE-A- 2 411 727**          **FR-A- 2 653 338**
**GB-A- 1 185 943**

EP 0 865 787 B1

## Description

This invention is concerned with a method of making controlled release compositions.

Many compositions for the controlled delivery of pharmaceuticals are known in the art. These include, for example, oral dosage forms, transdermal patches, preformed implants, topical gels, and the like.

Some of the above controlled delivery compositions use silicone based materials as matrices or membranes through which pharmaceutical agents are able to diffuse into the body at a controlled rate. Silicone based materials are desirable in these compositions since they are generally inert to the body.

One example of silicone based materials in controlled release compositions is provided in European publication number 465,744. This publication teaches the use of a multi-part formulation including an active agent, an Si-H containing polymer, a polymer having unsaturated groups bound to silicon, a catalyst and a hydrophilic component. This formulation is mixed and applied to the body where it cures and forms a controlled release gel.

The prior art methods such as those described in EP 465,744, however, have several disadvantages. For instance, in such methods the person utilising the formulation must be skilled so as to ensure adequate mixing of the appropriate amounts of component materials in the formulation and then applying the correct amount of the mixed formulation to the desired site before it gels. Similarly, such a method can be an inconvenience and messy for the user.

We have now discovered a simple method of making a controlled release composition which can avoid many of the above prior art problems.

The present invention provides in one of its aspects a method of making a controlled release composition comprising preparing a sprayable formulation comprising an active agent (A) and a vehicle (B), said vehicle comprising a curable silicone formulation comprising (I) a polysiloxane having Si-H bonds, (II) a polysiloxane having unsaturated hydrocarbon groups attached to silicon and (III) a hydrosilylation catalyst, wherein the component materials of vehicle (B) are separated into a plurality of containers to inhibit curing prior to spraying; and spraying the sprayable formulation onto the desired site, wherein said spraying causes mixing of the sprayable formulation and wherein said mixed sprayable formulation cures in situ on the desired site to form the controlled release composition.

By the above method, a sprayable formulation can be mixed and applied to a desired site by spraying where it cures to a controlled release composition in situ. The resultant controlled release composition is then capable of delivering an active agent (A) at a controlled rate.

As used in this specification, the 'formulations' are the mixtures of uncured component materials which are precursors to the final, cured, controlled release 'compositions'.

The active agent (A) used in the present invention is generally not critical. It can comprise any solid or liquid material which can be bound in the controlled release composition and subsequently released at the desired rate. The active agent (A) should also not interfere with the curing of the silicone formulation to an unacceptable extent. Suitable active agents (A) include cosmetics and therapeutic or diagnostic materials.

The invention is especially applicable to those therapeutic and diagnostic active agents (A) which benefit from delivery over a period of time at a controlled rate. For example, it is known that for certain drugs it is desirable to have the quantity of drug in the animal body continuously remain within a therapeutic window. By tailoring a formulation according to the invention, it is possible to provide controlled release compositions which deliver the drugs at rates which keep their concentrations in the body within their therapeutic windows.

Therapeutic active agents (A) which may be employed include, for example, antibiotic, antiseptic, anti-inflammatory, hormones, anticancer agents, smoking cessation compositions, cardiovascular, histamine blocker, bronchodilator, analgesic, antiarrythmic, antihistamine, alpha-1 blocker, beta blocker, ACE inhibitor, diuretic, antiaggregant, sedative, tranquilliser, anticonvulsant, anticoagulant agents, vitamins, agents for treating gastric and duodenal ulcers, proteolytic enzymes, healing factors, cell growth nutrients, peptides and others. Specific examples of suitable therapeutic active agents (A) include penicillins, cephalosporins, tetracyclines, macrolides, epinephrine, amphetamines, aspirin, barbiturates, catecholamines, benzodiazepine, thiopental, codeine, morphine, procaine, lidocaine, sulphonamides, ticonazole, perbuterol, furosamide, prazosin, prostaglandins, salbutamol, indomethicane, diclofenac, glafenine, dipyridamole, and theophylline.

In addition to the therapeutic or diagnostic materials, active agents (A) could be cosmetics such as perfumes, deodorants or the like. Suitable cosmetics are known to those skilled in the art.

The proportion of the active agent (A) employed in the present invention is chosen in accordance with the concentration of the active agent (A) required in the controlled release composition to deliver the dosage required at the proposed delivery rate. This may vary within a wide range such as from 0.1 to about 70 weight percent, preferably 0.1 to 20 weight percent, of the final controlled release composition.

The vehicle (B) used in the present invention comprises silicones ('polysiloxanes') or silicone-based materials which cure to form binder matrices for the other components (e.g., the active agent (A)) of the invention (i.e., they contain or entrap such components).

The polysiloxanes used herein comprise those which have silicon-bonded hydrogen atoms (I) in combination with those that have silicon-bonded unsaturated hydrocarbon groups (II). These polysiloxanes undergo a hydrosilylation reaction in the presence of a catalyst (III) to yield chain extended or crosslinked gelled or elastomeric silicone products.

Suitable polysiloxanes having Si-H bonds (I) include those having units according to the general formula $R_pHSiO_{(3-p/2)}$ in which each R represents a monovalent hydrocarbon group containing 1 to 20 carbon atoms, such as alkyl (e.g., methyl, ethyl, propyl or butyl) or phenyl groups and $\underline{p}$ is 0, 1, or 2. It is preferred that each R represents a methyl group. It is also preferred that the terminal groups have the formula $R_3SiO_{1/2}$ where each R represents a methyl group.

The polysiloxanes having silicon bonded hydrogen atoms may also be copolymers comprising, for example, units $R_nSiO_{(4-n/2)}$ in which R is as referred to above, and $\underline{n}$ is 0, 1, 2 or 3. Mixtures of polysiloxanes having silicon-bonded hydrogen atoms can also be used herein.

Preferably the polysiloxane having silicon-bonded hydrogen atoms has from 0.1% to 5% by weight hydrogen atoms based on the weight of the polymer.

Suitable alkylhydrogen polysiloxanes include those comprising $CH_3HSiO$ units with or without the presence of $Me_2SiO$ units and having viscosities on the order of from about 1 to about 1000 $mm^2/s$, more preferably from about 5 to about 50 $mm^2/s$ at 25° C.

Suitable polysiloxanes having silicon-bonded unsaturated groups (II) are those with sufficient unsaturated groups for formation of the polymer network. For example, polysiloxanes having siloxane units according to the general formula $R_mR'SiO_{(3-m/2)}$ in which each R represents a monovalent hydrocarbon group having 1 to 20 carbon atoms such as alkyls (e.g., methyl, ethyl, propyl or butyl) or phenyl groups, $\underline{m}$ is 0, 1 or 2 and R' represents an aliphatically unsaturated group such as vinyl, allyl, hexenyl and cyclohexenyl or a group R"CH=CHR'", where R" represents a divalent aliphatic chain linked to the silicon atom and R'" represents a hydrogen atom or an alkyl group. Preferably, R is methyl.

The polysiloxanes having silicon-bonded unsaturated groups can also comprise copolymers having, for instance, units $R_nSiO_{(4-n/2)}$ in which R is as referred to above, and $\underline{n}$ is 0, 1, 2 or 3. Mixtures of polysiloxanes having silicon-bonded unsaturated groups can also be used herein.

Preferably, the polysiloxanes having silicon-bonded unsaturated groups have from 0.01% to 1% by weight of aliphatically unsaturated groups and a viscosity on the order of about 10 $mm^2/s$ to about 25000 $mm^2/s$ at 25° C. More preferably, their viscosity is in the range of 100 $mm^2/s$ to 2000 $mm^2/s$ at 25°C.

The catalysts (III) used in the present invention comprise those known in the art to facilitate the hydrosilylation reaction. These include, for example, platinum and rhodium materials. These catalysts may take any of the known forms such as platinum or rhodium deposited on carriers such as silica gel or powdered charcoal or other appropriate compounds such as platinic chloride, salts of platinum and chloroplatinic acids. A preferred material is chloroplatinic acid either as the commonly obtainable hexahydrate or the anhydrous form because of its easy dispersibility in organosilicon systems and its non-effect on colour of the mixture. Platinum or rhodium complexes may also be used e.g. those prepared from chloroplatinic acid hexahydrate and divinyltetramethyldisiloxane.

When the polysiloxanes and the catalyst of the invention are mixed, they cure at room temperature (20 $\pm$ 5° C) within 10 minutes or, more preferably, within five minutes or less. In order to achieve satisfactory cure it is important that the ratio of silicon-bonded hydrogen atoms of the polysiloxanes to all groups reactive therewith in the formulation (e.g., the unsaturated groups) is appropriate to effect the desired cure. The curing time is dependent on various factors including the type and proportion of other component materials present in the formulation.

The proportion of cured binder matrix derived from vehicle (B) in the controlled release composition may vary widely depending on the intended site of application and the use of the composition. For example, the controlled release compositions may contain from 30 to 99% by weight of such cured binder matrix.

The final controlled release composition can be in the form of a gel or an elastomer and it can have pores (e.g., foams) or it can be pore-free.

If it is desired to prolong the cure time, one may include in the formulation one of the known catalyst inhibitors such as cyclic polymethylvinylsiloxane compounds or an acetylenic alcohol e.g. methyl butynol but these are not generally preferred in a formulation according to the invention.

If foaming of the formulation is desired, it may be induced by, for example, including a polysiloxane having silicon-bonded hydroxyl groups which reacts with the polysiloxane having silicon-bonded hydrogen atoms as more fully described, for example, in U.S. 4,026,845.

Alternatively, water, an aliphatic alcohol (for example a primary aliphatic or araliphatic alcohol such as a lower aliphatic monofunctional alcohol having up to 12 carbon atoms (e.g. ethanol, n-propanol, or benzyl alcohol) or a volatile blowing material can be included in the formulation as more fully described, for example, in U.S. 4,550,125. Preferred foamable formulations include compounds having silicon-bonded or carbon-bonded hydroxyl groups which foam and cure in presence of a platinum catalyst according to the scheme:

$$\#SiH + HOQ \longrightarrow \#SiOQ + H_2.$$

The group Q may be, for example, an aliphatic group or a polysiloxane having one or more reactive hydroxyl groups so that by virtue of the plurality of silicon-bonded or carbon-bonded hydroxyl groups the hydrogen evolved as a gas serves to form cells within the developing network of interconnected polysiloxane chains.

If desired the formulation may also contain other additional ingredients such as fillers (which may be, for example, opaque to X rays or other diagnostic radiation - especially useful for elastomeric products), colorants, coloured indicators, diluents, extender such as silicone fluids, silicone resins, excipients employed in pharmacy, compounds intended to perform as pH buffers in controlling the environment immediately in and around the formulation, stabilisers or surfactants for cellular formulations such as fluorinated silicones, processing aids such as cyclic or linear polydiorganosiloxanes, bioadhesive materials, and hydrophilic, modulating and swellable components or polymers as set forth in EP Publication 465,744.

One particularly advantageous optional ingredient in the formulation of the present invention is a diluent. Such diluents are often necessary to decrease the viscosity of the formulation sufficiently to permit spraying. Examples of diluents include silicon containing diluents such as hexamethyldisiloxane, octamethyltrisiloxane, and other short chain linear siloxanes, cyclic siloxanes such as octamethylcyclotetrasiloxane and decamethylcyclopentasiloxane, organic diluents such as alkanes, alcohols, or ketones or any other material which can dilute the formulation without adversely affecting any of the component materials of the formulation or the curing time.

The above diluents are often used in amounts of between about 20 and 90 wt % of the vehicle (B). On application, however, the vehicle often volatilizes leaving the other component materials on the desired site.

Since mixing of the component materials in vehicle (B) causes curing at room temperature, these component materials are stored in a plurality of containers prior to use to inhibit curing prior to spray. For instance, one container could contain the catalyst and a second could contain the polysiloxanes. Alternatively, the catalyst could be mixed with one of the siloxanes in one container and the other siloxane could be in a second container.

Each of the additional components in the formulation are put in the container which is most desirable depending on factors such as stability, viscosity, and interactions. Often, however, it is preferable to include the active agent (A) in only one of the parts of the formulation in order to preserve its effectiveness. Similarly, it often desirable to put a diluent in both containers to aid in spraying.

According to the method of the invention, the sprayable formulation comprising the vehicle (B), the active agent (A) and any other optional components are sprayed onto the desired site in a manner which causes mixing of the component materials from the several containers and dispensing of the formulation. The formulation cures after being applied and results in a controlled release composition. Preferably, the sprayable formulations are sprayed on a biological surface including, but not limited to animal bodies (e.g., human or other animal).

The spraying herein is performed by conventional techniques known in the art. For instance, the sprayable formulation can be sprayed by a pump sprayer which mechanically pumps the formulation from the containers. Similarly, the sprayable formulation can be sprayed by a forced spray device in which the formulation is forced from the container by a propellant as is known in the art. Conventional propellants such as air or hydrocarbons will function herein as long as they don't adversely interfere with the formulation or its curing.

Whichever of the above means of spraying is chosen, the formulation is generally forced through a nozzle which can be directed at the desired site for applying. The nozzle can merely direct a stream of the formulation at the desired site or it can create droplets of the formulation by techniques known in the art.

Spraying of the sprayable formulation must also result in a mixing of the component materials in the formulation from the separate containers. This, too, can occur by techniques known in the art. For instance, a mixing chamber can be built into the spraying apparatus such that as the component materials are drawn or forced out of their separate containers they are mixed prior to being fed through the nozzle. Similarly, the component materials can be mixed as they are forced though the nozzle. In yet another embodiment, the component materials of the separate containers can be forced through separate nozzles and the separate streams of component materials then mix either in the stream or as they contact the desired site. In still another embodiment, the component materials of the separate containers can be sprayed sequentially and then mix as they contact the desired site.

Depending on the active agent (A), the spraying mechanism often must be capable of delivering a controlled amount of the sprayable formulation such that the appropriate dose of the active agent (A) is delivered. Mechanisms for controlling the amount of formulation delivered by a spray are known in the art. For instance, the mechanical pump can be designed to deliver a controlled quantity of formulation with a metering valve. Similarly, the amount of propellant used with an activation can be controlled such that discrete quantities of formulation are expelled.

As noted above, when the formulation is mixed, it cures rapidly at room temperature (within 10 minutes). If used on an animal, this can minimise the amount time necessary to keep the area immobile while curing takes place.

The present invention offers numerous advantages over the prior art. The method described herein allows for the simple dispensing of the controlled release composition. As such, a skilled practitioner is not required for application. Likewise, the formulation chosen enables controlled release compositions to be formed by simple and easily controlled methods in situ. Moreover, the controlled release composition can be formed into a wide variety of shapes and have selected combinations of properties (e.g. bioadhesion, release rate and release profile). Similarly, the formulations and processes described herein don't involve severe conditions (e.g. high temperatures or pressures) which might damage the active agents (A) used.

The formulation and controlled release composition herein are generally acceptable on many biological membranes. The controlled release composition may be formed on intact or damaged skin or in a natural or artificial cavity of the body. The cavity may be, for example, the ocular, buccal, nasal, aural, vaginal or rectal cavity or a cavity formed, for example, in a tooth or an open wound.

The compositions may be formulated to give a moderate to rapid release of active agent (A). The drug delivery profile of compositions according to the invention may be predetermined by appropriate selection of the types and proportions of component materials and ingredients used.

It is a further advantage of the present invention that the controlled release compositions can have many physical properties from gels to elastomers so that they are able to withstand many of the pressures exerted during normal activities of a patient.

To clarify the invention, Examples follow which illustrate the methods of the invention. Unless indicated, all parts are by weight and all viscosities are at 25°C.

Example 1

In one container was mixed 43 parts of dimethyl vinyl end-blocked polydimethylsiloxane fluid (average dp = 147, molecular weight = 11,000, viscosity at 25 °C = 4.5 g/(cm x sec)), 2 parts hydrogen end blocked siloxane (average dp = 40, molecular weight = 2560, viscosity = 30 mm$^2$/s at 25°C), 50 parts hexamethyldisiloxane (dp = 2, molecular weight = 162, viscosity = 0.65 mm$^2$/s at 25°C) and 5 parts ketoprofene.

In a second container was mixed 90 parts hexamethyldisiloxane as in the first container and 10 parts of a platinum catalyst complex (0.6 wt. % platinum).

These component materials were packaged into separate containers and the dose was delivered via a conventional mechanical metering valve. The 2 parts were sprayed in sequence and the resultant formulation cured to a controlled release composition film in 174 seconds.

Example 2

In one container was mixed 43 parts of dimethyl vinyl end-blocked polydimethylsiloxane fluid as in Example 1, 2 parts hydrogen end blocked siloxane as in Example 1, 50 parts hexamethyldisiloxane as in Example 1 and 5 parts diphenhydramine HCl.

In a second container was mixed 90 parts hexamethyldisiloxane as in Example 1 and 10 parts of a platinum catalyst complex as in Example 1.

These component materials were packaged and sprayed as in Example 1. The resultant formulation cured to a controlled release composition film in 270 seconds.

Example 3

In one container was mixed 43 parts of dimethyl vinyl end-blocked polydimethylsiloxane fluid as in Example 1, 2 parts hydrogen end blocked siloxane as in Example 1, 50 parts hexamethyldisiloxane as in Example 1 and 5 parts 17 beta oestradiol.

In a second container was mixed 90 parts hexamethyldisiloxane as in Example 1 and 10 parts of a platinum catalyst complex as in Example 1.

These component materials were packaged and sprayed as in Example 1. The resultant formulation cured to a controlled release composition film in 204 seconds.

Example 4

In one container was mixed 29 parts of dimethyl vinyl end-blocked polydimethylsiloxane fluid as in Example 1, 2 parts hydrogen end blocked siloxane as in Example 1, 50 parts hexamethyldisiloxane as in Example 1, 5 parts ketoprofene and 14 parts ethanol.

In a second container was mixed 90 parts hexamethyldisiloxane as in Example 1 and 10 parts of a platinum catalyst complex as in Example 1.

These component materials were packaged and sprayed as in Example 1. The resultant formulation cured to a controlled release composition film in 90 seconds.

Example 5

In one container was mixed 43 parts of dimethyl vinyl end-blocked polydimethylsiloxane fluid as in Example 1, 2 parts hydrogen end blocked siloxane as in Example 1, 50 parts hexamethyldisiloxane as in Example 1, 5 parts diphenhydramine HCl and 14 parts ethanol.

In a second container was mixed 90 parts hexamethyldisiloxane as in Example 1 and 10 parts of a platinum catalyst complex as in Example 1.

These component materials were packaged and sprayed as in Example 1. The resultant formulation cured to a controlled release composition film in 210 seconds.

Example 6

In one container was mixed 29 parts of dimethyl vinyl end-blocked polydimethylsiloxane fluid as in Example 1, 2 parts hydrogen end blocked siloxane as in Example 1, 50 parts hexamethyldisiloxane as in Example 1, 5 parts ketoprofene and 14 parts ethanol.

In a second container was mixed 90 parts hexamethyldisiloxane as in Example 1 and 10 parts of a platinum catalyst complex as in Example 1.

These component materials were packaged and sprayed as in Example 1. The resultant formulation contained 2 mg ketoprofene.

## Claims

1. A method of making a controlled release composition comprising:

   preparing a sprayable formulation comprising an active agent (A) and a vehicle (B), said vehicle (B) comprising a curable silicone formulation comprising (I) a polysiloxane having Si-H bonds, (II) a polysiloxane having unsaturated hydrocarbon groups attached to silicon and (III) a hydrosilylation catalyst, wherein the components materials of vehicle (B) are separated into a plurality of containers to inhibit curing prior to spraying; and
   spraying the sprayable formulation onto the desired site, wherein said spraying causes mixing of the sprayable formulation and wherein said mixed sprayable formulation cures in situ on the desired site to form the controlled release composition.

2. The method according to Claim 1 wherein the active agent (A) is selected from the group consisting of cosmetics, antibiotics, antiseptics, hormones, anticancer agents, smoking cessation compositions antiinflammatories, cardiovasculars, histamine blockers, bronchodilators, analgesics, antiarrythmics, alpha-1 blockers, beta blockers, ACE inhibitors, diuretics, antiaggregants, sedatives, tranquillisers, anticonvulsants, anticoagulants, vitamins, agents for treating gastric and duodenal ulcers, proteolytic enzymes, healing factors and peptides.

3. The method according to any of the previous claims wherein active agent (A) is present in the controlled release composition at a concentration of from 0.1 to 70 parts by weight based on the weight of the controlled release composition.

4. The method according to any of the previous claims wherein vehicle (B) comprises 10 to 80 parts by weight of a mixture of (I), (II), and (III) and 90 to 20 parts by weight of a diluent.

5. The method according to Claim 4 wherein the diluent is a volatile siloxane.

6. The method according to claim 5 wherein the diluent is hexamethyldisiloxane.

7. The method according to any of the previous claims wherein the sprayable formulation also contains a filler.

8. The method according to any of the previous claims wherein the sprayable formulation also contains ingredients selected from the group consisting of pH buffers, hydrophilic materials, modulating materials, and swellable materials.

9. The method according to any of the previous claims wherein the sprayable formulation is sprayed on a biological membrane.

## Patentansprüche

1. Ein Verfahren zur Herstellung einer Zusammensetzung zur kontrollierten Freisetzung, umfassend:

   Herstellen eines sprühbaren Ansatzes, enthaltend einen aktiven Wirkstoff (A) und einen Trägerstoff (B), wobei der Trägerstoff (B) eine härtbare Siliconformulierung enthält, die (I) ein Polysiloxan mit Si-H-Bindungen, (II) ein Polysiloxan mit ungesättigten Kohlenwasserstoffgruppen, gebunden an Silicium, und (III) einen Hydrosilylierungskatalysator enthält, wobei die Komponenten der Materialien des Trägerstoffes (B) in einer Vielzahl von Behältern getrennt sind, um das Aushärten vor dem Sprühen zu verhindern; und
   Sprühen des sprühbaren Ansatzes auf die gewünschte Stelle, wobei das Sprühen das Mischen des sprühbaren Ansatzes bewirkt, und wobei dieser gemischte sprühbare Ansatz in situ auf der gewünschten Stelle aushärtet, um die Zusammensetzung für die kontrollierte Freisetzung zu bilden.

2. Ein Verfahren gemäß Anspruch 1, worin der aktive Wirkstoff (A) ausgewählt ist aus der Gruppe bestehend aus Kosmetika, Antibiotika, Antiseptika, Hormonen, Antikrebsmitteln, Zusammensetzungen, um das Rauchen aufzugeben, Antientzündungsmitteln, Herz- und Kreislaufmitteln, Histaminblockern, Bronchiodilatoren, Analgetika, Antiarythmika, α-1-Blockern, Betablockern, ACE-Inhibitoren, Diuretika, Antiaggregantien, Sedativa, Beruhigungsmitteln, Mittel gegen Krämpfe, Antikoagulantien, Vitaminen, Wirkstoffen zur Behandlung von Magen- und Darmkrebs, proteolytischen Enzymen, Heilungsfaktoren und Peptiden.

3. Das Verfahren gemäß einem der vorhergehenden Ansprüche, worin der aktive Wirkstoff (A) in der Zusammensetzung zur kontrollierten Freisetzung mit einer Konzentration von 0,1 bis 70 Gewichtsteilen, bezogen auf das Gewicht der Zusammensetzung zur kontrollierten Freisetzung, vorliegt.

4. Das Verfahren entsprechend einem der vorhergehenden Ansprüche, worin der Trägerstoff (B) 10 bis 80 Gewichtsteile einer Mischung von (I), (II) und (III) und 90 bis 20 Gewichtsteile eines Verdünnungsmittels enthält.

5. Das Verfahren gemäß Anspruch 4, worin das Verdünnungsmittel ein flüchtiges Siloxan ist.

6. Das Verfahren gemäß Anspruch 5, worin das Verdünnungsmittel Hexamethyldisiloxan ist.

7. Das Verfahren gemäß einem der vorhergehenden Ansprüche, worin der sprühbare Ansatz auch einen Füllstoff enthält.

8. Das Verfahren gemäß einem der vorhergehenden Ansprüche, worin der sprühbare Ansatz auch Bestandteile enthält, die ausgewählt sind aus der Gruppe bestehend aus pH-Puffern, hydrophilen Materialien, modulierenden Materialien und quellbaren Materialien.

9. Das Verfahren gemäß einem der vorhergehenden Ansprüche, worin der sprühbare Ansatz auf eine biologische Membran gesprüht wird.

**Revendications**

1. Procédé de préparation d'une composition à libération contrôlée, comprenant :

la préparation d'une formulation pulvérisable comprenant un principe actif (A) et un véhicule (B), ledit véhicule (B) comprenant une formulation siliconée durcissable comprenant (I) un polysiloxane ayant des liaisons Si-H, (II) un polysiloxane ayant des groupes hydrocarbonés insaturés fixés et (III) un catalyseur d'hydrosilylation, où les substances composant le véhicule (B) sont séparées dans une pluralité de récipients pour empêcher le durcissement avant la pulvérisation ; et
la pulvérisation de la formulation pulvérisable sur le site désiré, où ladite pulvérisation provoque le mélange de la formulation pulvérisable et où ladite formulation pulvérisable mélangée durcit sur place sur le site désiré pour former la composition à libération contrôlée.

2. Procédé selon la revendication 1, où le principe actif (A) est sélectionné dans le groupe constitué par les cosmétiques, les antibiotiques, les antiseptiques, les hormones, les agents anticancéreux, les compositions pour arrêter de fumer, les anti-inflammatoires, les cardio-vasculaires, les bloqueurs de l'histamine, les bronchodilatateurs, les analgésiques, les antiarythmiques, les alpha-1-bloquants, les bêta-bloquants, des inhibiteurs de l'ACE, les diurétiques, les antiagrégants, les sédatifs, les tranquillisants, les anticonvulsivants, les anticoagulants, les vitamines, les agents de traitement des ulcères gastroduodénaux, les enzymes protéolytiques, les facteurs de guérison et les peptides.

3. Procédé selon l'une quelconque des revendications précédentes, où le principe actif (A) est présent dans la composition à libération contrôlée en une concentration comprise entre 0,1 et 70 parties en poids par rapport au poids de la composition à libération contrôlée.

4. Procédé selon l'une quelconque des revendications précédentes, où le véhicule (B) comprend 10 à 80 parties en poids d'un mélange de (I), (II) et (III) et 90 à 20 parties en poids d'un diluant.

5. Procédé selon la revendication 4, où le diluant est un siloxane volatil.

6. Procédé selon la revendication 5, où le diluant est l'hexaméthyldisiloxane.

7. Procédé selon l'une quelconque des revendications précédentes, où la formulation pulvérisable contient également une charge.

8. Procédé selon l'une quelconque des revendications précédentes, où la formulation pulvérisable contient également des ingrédients sélectionnés dans le groupe constitué par des tampons de pH, des substances hydrophiles, des substances modulantes et des substances gonflables.

9. Procédé selon l'une quelconque des revendications précédentes, où la formulation pulvérisable est pulvérisée sur une membrane biologique.